Europäisches Patentamt

European Patent Office

Office européen des brevets

(19)

(11) Veröffentlichungsnummer: **0 004 370**
**B1**

(12) **EUROPÄISCHE PATENTSCHRIFT**

(45) Veröffentlichungstag der Patentschrift:
12.08.81

(51) Int. Cl.³: **A 61 N 5/06,** A 61 N 5/08

(21) Anmeldenummer: **79100840.2**

(22) Anmeldetag: **20.03.79**

(54) Bestrahlungsvorrichtung, insbesondere Heimsolarium.

(30) Priorität: **20.03.78 DE 2812129**
**10.05.78 DE 7814113 U**
**22.12.78 DE 2855747**
**14.03.79 DE 2910068**

(43) Veröffentlichungstag der Anmeldung:
**03.10.79 Patentblatt 79/20**

(45) Bekanntmachung des Hinweises auf die Patenterteilung:
**12.08.81 Patentblatt 81/32**

(84) Benannte Vertragsstaaten:
**BE CH DE FR GB IT NL SE**

(56) Entgegenhaltungen:
**DE-A-2 707 920**
**DE-U-7 239 096**
**DE-U-7 814 113**
**US-A-2 327 346**
**US-A-3 648 706**
**US-A-2 998 508**

(73) Patentinhaber: **Patent-Treuhand-Gesellschaft für elektrische Glühlampen mbH, Hellabrunner Strasse 1, D-8000 München 90 (DE)**

(72) Erfinder: **Jendrewski, Alfons, Rudolf-Wilke-Weg 12, D-8000 München 71 (DE)**
Erfinder: **Schlagheck, Norbert, Rothschwaiger Strasse 42, D-8080 Fürstenfeldbruck (DE)**
Erfinder: **Schröder, Gerd, Schrimpfstrasse 67, D-8035 Gauting (DE)**
Erfinder: **Ziegler, Friedrich, Leitenweg 85, D-8190 Wolfratshausen (DE)**

BUNDESDRUCKEREI BERLIN

## Bestrahlungsvorrichtung, insbesondere Heimsolarium

Die Erfindung betrifft eine Bestrahlungsvorrichtung, insbesondere Heimsolarium, mit einem Gehäuse, das mehrere — mindestens drei — jeweils mit einem Reflektor versehene UV- und/oder IR-Strahler enthält, die an einem Trägerteil des Gehäuses angebracht sind, wobei durch die Strahlungsrichtung eines oder mehrerer Strahler eine vertikale Hauptstrahlungsrichtung vorgegeben ist, und mit einer Haltevorrichtung, bestehend aus mindestens einem Haltearm, der an seinen Enden jeweils mit einem Gelenk versehen ist, wobei der Haltearm mittels des ersten Gelenkes am Gehäuse befestigt ist.

Derartige Bestrahlungsgeräte (Heimsolarien) werden zur Körperbestrahlung von Personen eingesetzt, und zwar sowohl im kosmetischen als auch im therapeutischen Bereich. Die bekannten Geräte sind entweder an der Raumdecke aufzuhängen oder an einer Wandhalterung befestigt, an der sie in vertikaler Richtung abklappbar sind. Die an der Raumdecke aufzuhängenden Heimsolarien sind allenfalls in einer Heimsauna zu verwenden, da in gewöhnlichen Schlaf- und Wohnzimmern ein wegen der erforderlichen festen Installierung ästhetisch recht unbefriedigender Eindruck entsteht. Das gleiche gilt für die an der Wand zu befestigenden abklappbaren Solarien, die zudem noch den Nachteil geringer Ausladung aufweisen.

Für den Gebrauch in Wohnräumen wurden deshalb sogenannte Standsolarien auf den Markt gebracht, die vertikal auf einem fahrbaren Stativ befestigt werden können. Da diese Solarien aber in horizontaler Richtung strahlen, muß sich der Benutzer während der Bestrahlung vor sie hinstellen oder -setzen, was als für ein Sonnenbad unbequem und unnatürlich empfunden wird.

Nach der US-A-3 648 706 ist eine Bestrahlungsvorrichtung bekannt, bei der mehrere jeweils mit einem Reflektor versehene IR-Strahler in flächenförmiger Anordnung innerhalb eines Gehäuses an einem Trägerteil angebracht sind und bei der eine Haltevorrichtung vorgesehen ist, bestehend aus zwei scherenartig angeordneten Haltearmen, die mittels Gelenken an dem genannten Trägerteil und an einem Traggerüst befestigt sind. Dabei verlaufen die Gelenkachsen der Haltearme im wesentlichen senkrecht zu der vertikalen Hauptstrahlungsrichtung der Strahler, wodurch die Lage des Strahlergehäuses in der Hauptstrahlungsrichtung veränderbar ist. Mit dieser Haltevorrichtung läßt sich hinsichtlich der zu bestrahlenden Person der Bestrahlungsabstand variieren. Dieser ist bei den verwendeten reinen IR-Strahlern nicht kritisch (im Gegensatz zu UV-Strahlern, bei denen hinsichtlich des Bestrahlungsabstandes genaue Vorschriften eingehalten werden müssen).

In der US-A-2 998 508 ist eine Versorgungs- und Beleuchtungseinrichtung für Krankenhausbetten beschrieben, bei der die beiden Leuchten, eine Lese- und eine Untersuchungsleuchte, an schwenkbaren Haltearmen befestigt sind. Die beiden Leuchten, die mittels Universalgelenken an den Haltearmen angebracht sind, können nahezu eine beliebige Strahlungsrichtung einnehmen. Die Haltearme dienen der universellen Positionierung der Leuchten (evtl. Versorgung zweier Krankenbetten). Bei einer solchen Beleuchtungsvorrichtung ist kein kritischer Abstand zum Patienten einzuhalten.

Des weiteren ist nach US-A-2 327 346 eine Bestrahlungsvorrichtung bekannt, bei der ein langgestreckter UV-Strahler — evtl. mit IR-Heizelementen — in einem Reflektorgehäuse angeordnet ist und bei der eine Haltevorrichtung vorgesehen ist, die entsprechend einem Ausführungsbeispiel aus zwei parallelen Haltearmen besteht, die mittels Gelenken am Reflektorgehäuse und an einem Tragrahmen befestigt sind. Diese Haltevorrichtung mit horizontal verlaufenden Gelenkachsen dient zum Schwenken des Reflektorgehäuses in einer vertikalen Ebene über den gesamten Winkelbereich von 360°, wodurch auch die Hauptstrahlungsrichtung des Strahlers mit Reflektor in der Vertikalebene über diesen Winkelbereich veränderbar ist, entsprechend einer beliebigen Stellung oder Lage der zu bestrahlenden Person.

Bei solchen Bestrahlungsgeräten wird insbesondere eine möglichst gleichmäßige Bestrahlungsstärke über die ganze zu bestrahlende Körperfläche angestrebt. Dies ist jedoch nicht bei allen bekannten Bestrahlungsgeräten erreicht. Mit einer Flächenanordnung der Strahler entsprechend dem DE-GM 7 109 480 konnten gute Ergebnisse erzielt werden. Bezüglich Bestrahlungsgeräten mit flächenförmiger Strahleranordnung wurden auch Vorschläge gemacht, durch die sich kompaktere Geräteabmessungen erhalten lassen. So werden — bei kleinerer Strahlerzahl — z. B. einige der Strahler mit ihren Reflektorachsen gegenüber der Bestrahlungsfläche geneigt angeordnet (DE-OS 2 356 272 und DE-GM 7 605 558). Es ist auch ein Bestrahlungsgerät mit Reihenanordnung der Strahler bekannt (DE-GM 7 239 096); bei diesem sind alle UV- und IR-Strahler mit ihren Reflektorachsen senkrecht zur Bestrahlungsfläche ausgerichtet.

Der Erfindung liegt die Aufgabe zugrunde, eine Bestrahlungsvorrichtung (Heimsolarium) zu schaffen, die sich platzsparend und einfach installieren läßt und die einfach in der Handhabung ist. Die Vorrichtung sollte eine Bestrahlung im Liegen erlauben und dabei insbesondere für den Gebrauch in Wohnräumen geeignet sein. Trotz kompakter Geräteabmessungen sollte sich eine gute Gleichmäßigkeit der Bestrahlungsstärke auf der zu bestrahlenden Körperoberfläche erreichen lassen. Durch eine entsprechende Bestrahlungsstärke auf der Bestrahlungsfläche soll eine kurze Bestrahlungszeit gewährleistet sein.

Diese Aufgabe ist bei einer Bestrahlungsvorrichtung (Heimsolarium) mit den im Oberbegriff des Hauptanspruchs genannten Merkmalen erfindungsgemäß dadurch gelöst, daß die Achsen der beiden Gelenke des Haltearmes im wesentlichen parallel zur Hauptstrahlungsrichtung verlaufen, wobei das zweite Gelenk an einer Halteplatte zum Befestigen der Bestrahlungsvorrichtung gelagert ist, und daß die Strahler innerhalb des Gehäuses in einer einzigen Reihe angeordnet sind. In einer besonders vorteilhaften Ausführung ist die vertikale Hauptstrahlungsrichtung durch die Strahlungsrichtung eines mittleren Strahlers oder einer mittleren Strahlergruppe vorgegeben und die mit diesem mittleren Strahler bzw. dieser mittleren Strahlergruppe in einer Reihe angeordneten außermittigen Strahler sind mit ihren Reflektorachsen gegenüber der Hauptstrahlungsrichtung geneigt. Vorzugsweise liegen die Reflektorachsen aller Strahler im wesentlichen in einer Ebene und die Neigung der Reflektorachsen — bei den außermittigen Strahlern — stimmt mit der Richtung überein, in der der betreffende Strahler oder die Strahlergruppe gegenüber dem mittleren Strahler oder der mittleren Strahlergruppe versetzt ist.

Der die Bestrahlungsvorrichtung tragende Haltearm ist vorteilhaft — über das erste Gelenk — an einem Ende des Strahlergehäuses angebracht. Falls die Befestigung an der Oberseite des Gehäuses vorgenommen ist, ist der Haltearm länger als das Gehäuse. Der Haltearm kann auch an der Rückseite des Strahlergehäuses befestigt sein, er ist dann kürzer als das Gehäuse. Bei all diesen Ausführungen können die Bedienungshandhaben für den Strahlerbetrieb sowohl im Strahlergehäuse integriert sein, als auch auf der Wandhalterung (Halteplatte) oder direkt neben dieser in einem separaten Gehäuse angeordnet sein. Der schwenkbare Haltearm erlaubt ein einfaches Abrücken des Gerätes von der Wohnungswand, an der die Halteplatte befestigt ist. Das Gerät läßt sich ohne Mühe in verschiedene Stellungen bezüglich der Wand bringen. Im voll ausgeschwenkten Zustand können Haltearm und Strahlergehäuse — in etwa eine Gerade bildend — senkrecht zur Wohnungswand ausgerichtet sein. Nach dem Bestrahlungsvorgang kann die Bestrahlungsvorrichtung mittels des Haltearmes wieder an die Wand geschwenkt und somit aufgeräumt werden. Ein mit einer solchen Haltevorrichtung versehenes Bestrahlungsgerät ist insbesondere für die Bestrahlung von waagerecht liegenden Personen geeignet.

Bei schwereren Gehäusen sind vorteilhaft zwei Haletarme vorgesehen, die jeweils an den Enden des Strahlergehäuses an dessen Ober- oder Rückseite befestigt sind. Die einzelnen Haltearme sind in ihrer Länge unterteilt und die zusammengehörigen Teilarme durch ein (mittleres) Gelenk miteinander verbunden, dessen Achse zu den übrigen Gelenkachsen parallel liegt. Bei dieser Anordnung ist eine gleichmäßige Verteilung des Gehäusegewichtes erreicht. Die Teilarme sind jeweils kürzer als die halbe Länge des Strahlergehäuses. Bei Anordnung an der Gehäuserückseite sind die Haltearme in Richtung der Gelenkachsen breiter und quer zu den Gelenkachsen schmaler ausgebildet. An der Oberseite befestigt, sind die Haltearme in Richtung der Gelenkachsen schmaler und quer zu den Gelenkachsen breiter; die Breite der Haltearme entspricht in etwa der Breite der Gehäuseoberseite. Die Bedienungshandhaben für den Strahlerbetrieb können in einem separaten Gehäuse untergebracht sein; dieses befindet sich z. B. — falls die Haltearme an der Rückseite des Gehäuses angebracht sind — beim eingeschwenkten Zustand des Strahlergehäuses zwischen den mittleren Gelenken der Haltearme, die ihrerseits gegen die Mitte des Strahlergehäuses geschwenkt sind.

Der Neigungswinkel der Strahlerreflektoren wird weitestgehend von der gewählten Strahleranzahl und der Größe der Bestrahlungsvorrichtung, dem dadurch bedingten Abstand der Strahler voneinander, und dem Abstand der Bestrahlungsvorrichtung von der zu bestrahlenden Körperfläche bestimmt; er beträgt jeweils 10° bis 30°. Bei einer Vorrichtung für Ganzkörperbestrahlung wird eine Bestrahlungsfläche von 0,6 × 1,8 m zugrunde gelegt.

Es hat sich als günstig erwiesen, den Neigungswinkel der Reflektoren aller geneigt angeordneten Strahler gleich zu wählen. Hierdurch ist für den Bestrahlungsabstand, bei dem noch eine gleichmäßige Strahlungsverteilung erreicht wird, ein gewisser Bereich gegeben. Zur Gleichmäßigkeit der Bestrahlung trägt auch ein gleicher Abstand zwischen benachbarten Strahlern bei. Eine besonders vorteilhafte Bestrahlungsvorrichtung für Ganzkörperbestrahlung besteht aus fünf Strahlern, drei UV/IR-Mischstrahlern und zwei IR-Wärmestrahlern. Diese sind abwechselnd angeordnet, so daß sich in der Reihenmitte ein UV/IR-Mischstrahler befindet. Benachbarte Strahler haben einen Abstand von etwa 25 cm voneinander — gemessen zwischen den Mitten der Strahlungsaustrittsflächen. Der Neigungswinkel der Strahlerreflektoren, die sich außerhalb der Reihenmitte befinden, beträgt jeweils etwa 15°. Der in der Mitte befindliche Strahler ist senkrecht zum Trägerteil ausgerichtet; er bestimmt die Hauptstrahlungsrichtung. Bei dieser Anordnung erhält man eine günstige Strahlungsverteilung auf der Bezugsfläche bei einem Bestrahlungsabstand von 1 bis 1,4 m. Bei einer noch kompakteren Anordnung beträgt der Abstand benachbarter Strahler etwa 20 cm; die geneigt angeordneten Strahler haben einen Neigungswinkel von etwa 20° (als Bestrahlungsabstand ist ebenfalls 1 bis 1,4 m günstig). Wird der Abstand aufeinanderfolgender Strahler vergrößert, z. B. auf 30 cm, ist der Neigungswinkel der außermittigen Strahler kleiner zu wählen, er beträgt dann etwa 10°.

Wird der Bestrahlungsabstand sehr klein gewählt, etwa ≤1 m (bei Anordnungen mit einem äquidistanten Strahlerabstand zwischen 20 und 30 cm), um z. B. eine höhere Bestrahlungsstärke zu erreichen, sind — bei den außermittigen Strahlern — die äußeren Strahlerreflektoren gegenüber den weiter innenliegenden stärker zu neigen. So ist auch bei kleineren Bestrahlungsabständen eine verhältnismäßig gute Gleichmäßigkeit in der Bestrahlung gewährleistet. Um trotzdem im Bestrahlungsabstand variabel zu sein, können die äußeren Strahler mittels einer Rastvorrichtung in ihrem Neigungswinkel gegenüber dem Trägerteil veränderlich einstellbar sein. Es lassen sich unterschiedliche Neigungswinkel bei den Strahlerreflektoren natürlich auch fest vorgeben — mit zunehmendem Abstand der Strahler von der Reihenmitte größer werdende Neigungswinkel.

Neben Anordnungen, bei denen UV- und IR-Strahler alternierend angebracht sind, lassen sich auch Bestrahlungsvorrichtungen schaffen, bei denen die Reihenanordnung nur aus UV-Strahlern (bzw. UV/IR-Mischstrahlern) oder nur aus IR-Strahlern besteht. Für die Neigungswinkel und die Abstände der Strahlerreflektoren gelten dann die bereits gemachten Ausführungen. Es ist deshalb zweckmäßig, wenn sich die einzelnen Strahlertypen austauschen lassen. So läßt sich insbesondere die UV-Bestrahlungsstärke variieren, die am höchsten ist, wenn die Reihenanordnung nur UV-Strahler enthält. Eine Bestrahlungsvorrichtung, die nur IR-Wärmestrahler aufweist, ist vor allem im therapeutischen Bereich vorteilhaft einsetzbar.

Das die UV- und/oder IR-Strahler tragende — im wesentlichen ebene — Teil (Trägerteil) ist Bestandteil eines die Strahler aufnehmenden Gehäuses, z. B. die Gehäuseabdeckung, an der die Strahlerfassungen (im Innern des Gehäuses liegend) befestigt sind. Ein besonders kompaktes Bestrahlungsgerät ergibt sich, wenn die Längs- und Schmalseiten des Gehäuses die Strahlerreihe eng umgeben. Die Gehäusewände können dabei von den breitesten Abmessungen der Strahler, den Strahlungsaustrittsflächen (z. B. Strahlerkuppen), ausgehend z. B. senkrecht zum langgestreckten und ebenen Trägerteil hochgezogen sein oder sie können in ihrer Form den Strahlern angepaßt verlaufen und mit dem Trägerteil verbunden sein. Die Strahlungsaustrittsflächen der UV- und IR-Strahler sind von Gehäuseteilen freigehalten.

Anhand der folgenden Figuren sind mehrere erfindungsgemäße Ausführungsbeispiele erläutert. Es zeigt

Fig. 1 eine Ausführungsform der erfindungsgemäßen Bestrahlungsvorrichtung (insbesondere die Schwenkarmanordnung) in perspektivischer Ansicht — in Ruhestellung,

Fig. 2 den Gegenstand von Fig. 1 mit erfindungsgemäßer Strahleranordnung — in einer ersten Arbeitsstellung,

Fig. 3 den Gegenstand von Fig. 1 in einer zweiten Arbeitsstellung,

Fig. 4 eine Draufsicht auf eine weitere Ausführungsform (insbesondere der Schwenkarmanordnung) in einer Arbeitsstellung und in Ruhestellung,

Fig. 5 eine Draufsicht auf eine weitere Ausführungsform (insbesondere der Schwenkarmanordnung) in Arbeitsstellung,

Fig. 6 den Gegenstand von Fig. 5 in Ruhestellung,

Fig. 7 eine Draufsicht auf eine weitere Ausführungsform (insbesondere der Schwenkarmanordnung) in Arbeitsstellung,

Fig. 8 eine Seitenansicht einer Ausführungsform der erfindungsgemäßen Strahleranordnung — teilweise im Schnitt,

Fig. 9 den Gegenstand von Fig. 8 in einer von der Unterseite vorgenommenen Draufsicht,

Fig. 10a bis 13a den Gehäuseaufbau verschiedener Ausführungsformen im Schnitt,

Fig. 10b bis 13b denselben Gehäuseaufbau jeweils in perspektivischer Darstellung.

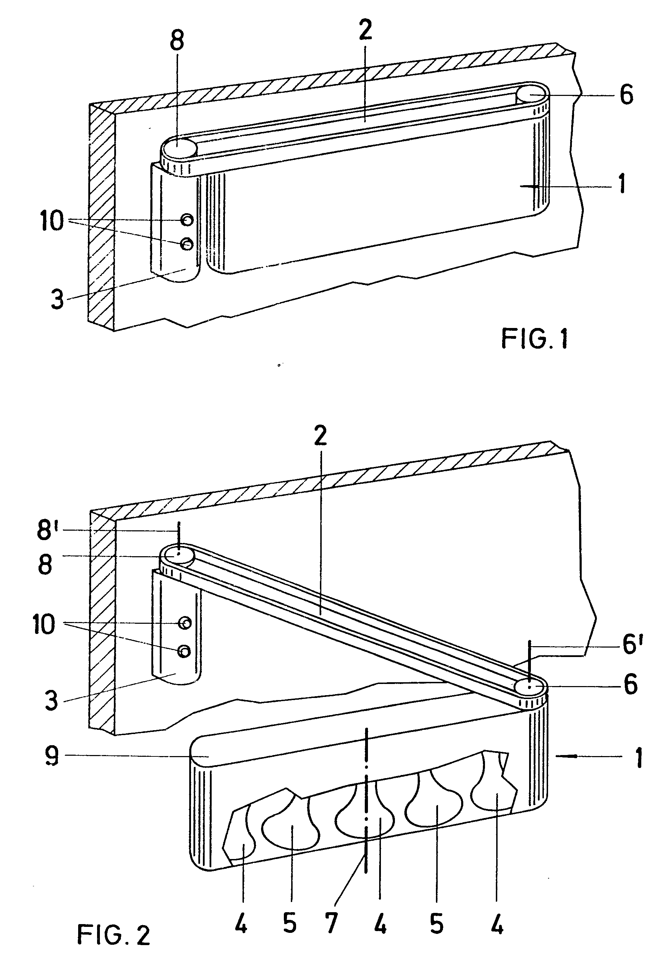

Die Fig. 1 bis 3 zeigen verschiedene Stellungen einer erfindungsgemäßen Ausführungsform. Diese besteht aus einem Gehäuse 1, das die Strahler enthält, einem Haltearm 2 und einer Wandhalterung 3. Um kompakte Geräteabmessungen zu erreichen, sind die Strahler — UV/IR-Mischstrahler 4 und IR-Wärmestrahler 5 — in einer Reihe angeordnet (Fig. 2). Der Haltearm 2 ist mit dem Gehäuse 1 durch ein Gelenk 6 schwenkbar verbunden. Der Haltearm 2 ist länger als das Gehäuse 1. Die Drehachse 6' des Gelenkes 6 liegt in der Hauptstrahlungsrichtung 7, die durch den mittleren UV/IR-Strahler 4 vorgegeben ist. Am anderen Ende trägt der schwenkbare Haltearm 2 ein weiteres Gelenk 8, und ist damit in der Halterung 3 gelagert. Diese Halterung 3 ist mittels geeigneter Verbindungsmittel an einer vertikalen Fläche, beispielsweise einer Wand oder Säule, befestigbar. Die Drehachse 8' des Gelenkes 8 liegt parallel zur Drehachse 6' des Gelenkes 6 und damit ebenfalls in der Hauptstrahlungsrichtung 7. Die Gehäuseoberseite 9 bildet gleichzeitig das Trägerteil für die Strahler 4, 5. Die Hauptstrahlungsrichtung 7 verläuft senkrecht zum Trägerteil. An der Halterung 3 sind Bedienungshandhaben 10 für die Strahler 4, 5 angeordnet.

Die Bestrahlungsvorrichtung ist an einer senkrechten Fläche durch Befestigen der Halterung 3 mit an sich geläufigen Mitteln, z. B. Schrauben und Dübeln, montiert; dabei sind die Achsen 6' und 8' parallel zur Befestigungsfläche ausgerichtet. Die für die elektrische Installation erforderlichen Kabel laufen im Gehäuseinnern bzw. im Innern des Haltearmes 2 und der Halteplatte 3 und sind daher nicht sichtbar.

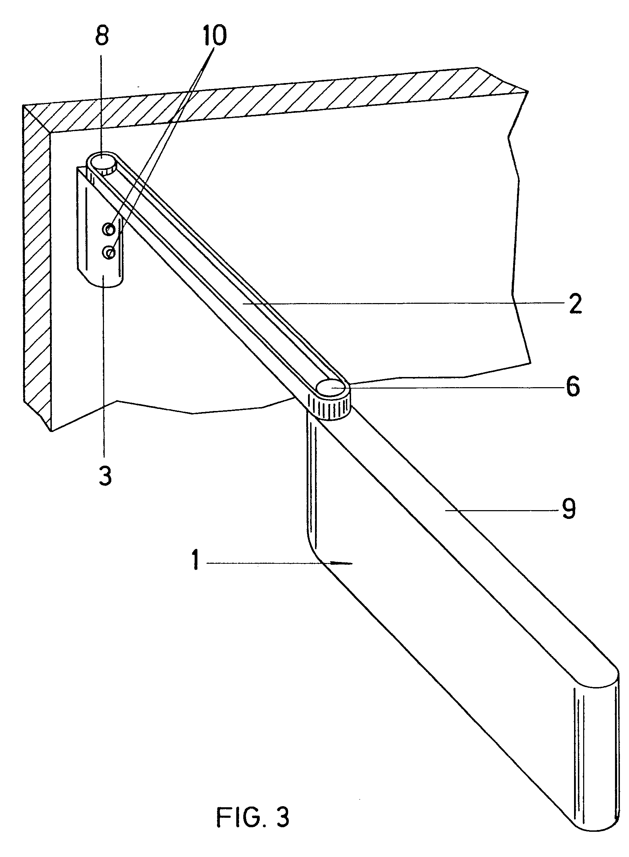

Das Gerät kann, wie aus den Figuren ersichtlich, in verschiedene Stellungen geschwenkt werden, nämlich in die Ruhestellung (Fig. 1) und in variable Arbeitsstellungen (Fig. 2 und 3). In der Ruhestellung liegt das Gerät an der Wand an und nimmt wenig Platz ein. Gleichzeitig werden die an der Oberseite 9 des Gehäuses 1 angeordneten Lüftungsschlitze (nicht dargestellt), die zur Kühlung beim Betrieb dienen, in der Ruhestellung vom Haltearm 2 abgedeckt, so daß kein Staub eindringen kann. Die variablen Arbeitsstellungen des Gerätes ermöglichen vielfältigen Einsatz. So ist mit einer einzigen Geräteausführung sowohl eine Befestigung am Kopfende als auch an einer Seite einer Liege möglich. Des weiteren läßt sich das Gerät auch am Kopfende eines Doppelbettes montieren. Dieser Platz ist bei den üblichen Wohungseinrichtungen besonders geeignet. Es kann dann sowohl das eine als auch das andere Bett durch Verschwenken von Haltearm 2 und Gehäuse 1 bestrahlt werden. Es versteht sich von selbst, daß die Gelenke 6 und 8 sowie der Haltearm 2 aber wenigstens so stabil ausgelegt werden müssen, daß das Gewicht des Gehäuses 1 sicher getragen wird.

Die Bedienungshandhaben 10 sind in jeder Stellung zugänglich. Da sie unmittelbar an der Wandhalterung 3 angebracht sind, kann bei ihrem Gebrauch das Gerätegehäuse 1 und der Haltearm 2 nicht aus Unachtsamkeit zu stark belastet werden. Ferner brauchen sie nicht gegen die sich im Gehäuse bei Betrieb entwickelnde Wärme abgeschirmt zu werden.

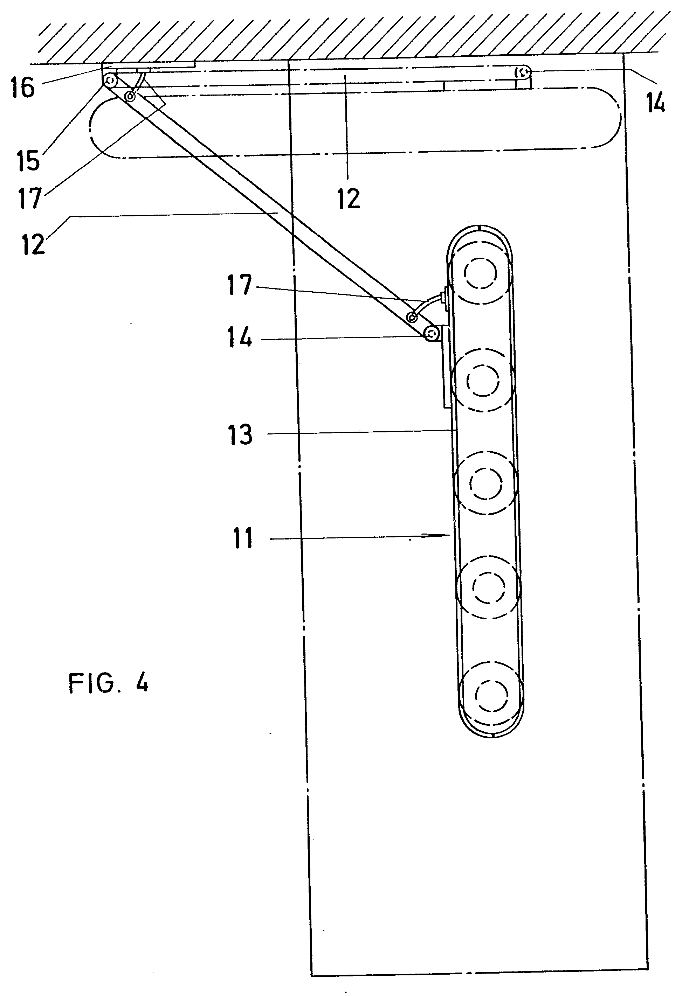

Fig. 4 zeigt eine weitere Ausführungsform des Gegenstandes der Erfindung von oben gesehen, und zwar in Ruhestellung (gestrichelt) und in Arbeitsstellung (durchgezogen). Das Gehäuse 11 kann wie vorher beschrieben ausgebildet sein. Der Haltearm 12 ist hier aber nicht an der Oberseite, sondern an der rückwärtigen Seitenfläche (Rückseite) 13 des Gehäuses 11 befestigt. Die Gelenke 14 und 15 verbinden das Gehäuse 11 und die Halterung 16 über den Haltearm 12. Bei dieser Ausführungsform ist letzterer kürzer als das Gehäuse 11, so daß er in Ruhestellung hinter demselben verborgen liegt. Das Stromkabel 17 ist zwar im Haltearm 12, nicht aber durch die Gelenke 14 und 15 geführt und somit in Arbeitsstellung teilweise sichtbar.

Der Haltearm 12 dieser Ausführungsform läßt sich als vertikal ausgerichtete Platte (bis zu einer Breite entsprechend der Höhe des Leuchtengehäuses) äußerst verwindungssicher gestalten, was die Verwendung auch schwererer Leuchtengehäuse erlaubt. Hierbei können die Bedienungshandhaben ohne weiteres auch am Gehäuse 11 angebracht sein. Um den Arbeitsbereich zu erweitern, kann der Schwenkarm auch teleskopisch ausgebildet sein.

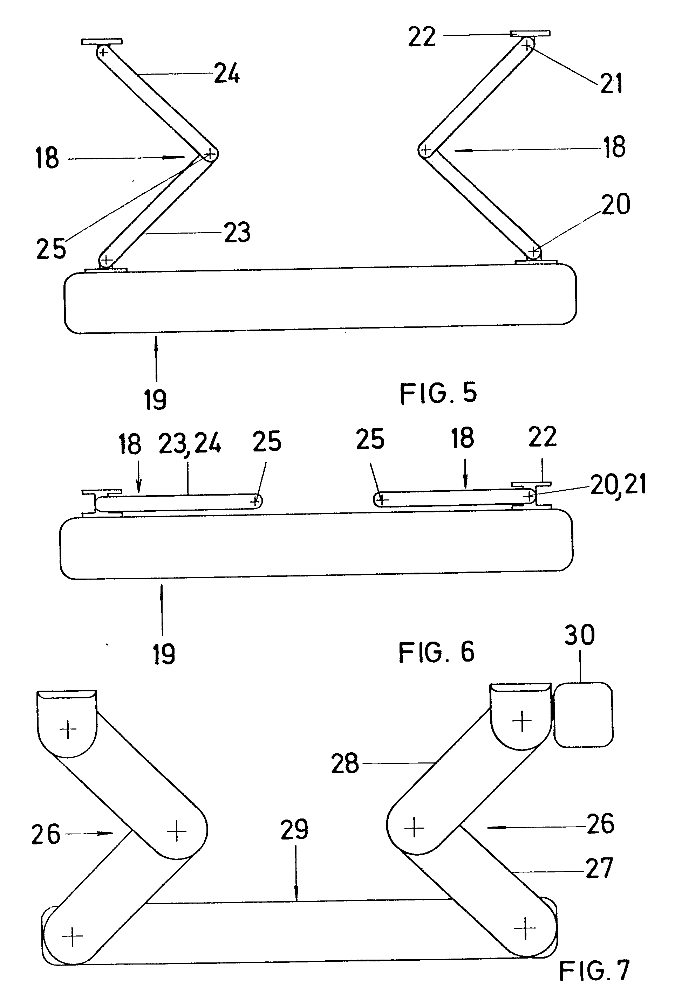

Besonders schwere Geräte sind mit einer Haltevorrichtung gemäß den Fig. 5 bis 7 ausgestattet. Es sind jeweils zwei Haltearme 18 vorgesehen, die z. B. an der Rückseite des Gehäuses 19 angebracht sind (Fig. 5 und 6). Die Haltearme 18 sind über Gelenke 20 mit dem Gehäuse 19 schwenkbar verbunden. Weitere Gelenke 21 verbinden die Haltearme 18 mit der Wandhalterung 22 (Halteplatten). Die Haltearme 18 sind in ihrer Länge in die Teilarme 23 und 24 unterteilt, die durch ein mittleres Gelenk 25 miteinander verbunden sind. Die Hauptstrahlungsrichtung der Strahler liegt senkrecht zur Zeichenebene. Entsprechend sind die Gelenkachsen der Gelenke 20, 21, 25 ausgerichtet. In Fig. 5 ist das Gerät in Arbeitsstellung gezeigt — teilweise ausgeschwenkt; in Fig. 6 befindet sich das Gerät in Ruhestellung, die Teilarme 23, 24 sind jeweils übereinanderliegend angeordnet. Bei dieser Ausführung sind die Bedienungshandhaben für den Strahlerbetrieb im Gehäuse 19 integriert. Ebenso können die Handhaben aber auch in einem separaten Gehäuse untergebracht sein, das sich in Ruhestellung des Gerätes (Fig. 6) z. B. an der Wand zwischen den Gelenken 25, 25 befindet (nicht dargestellt).

In Fig. 7 sind die Haltearme 26 (Teilarme 27 und 28) an der Oberseite des Gehäuses 29 angebracht. Das Gerät befindet sich in Arbeitsstellung. Die Bedienungshandhaben sind im separaten Gehäuse 30 angeordnet.

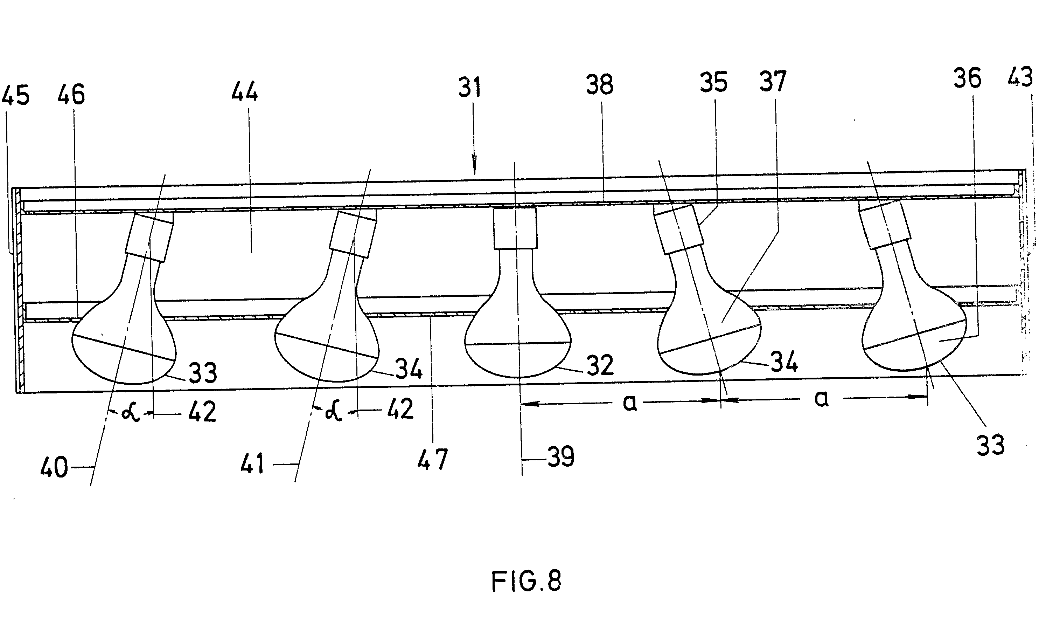

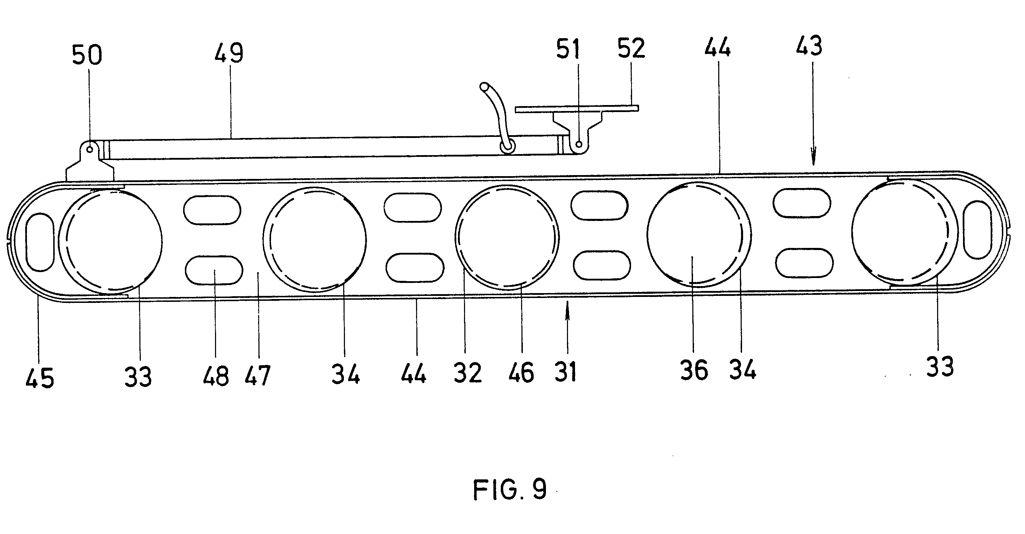

Die Fig. 8 und 9 zeigen ein Ausführungsbeispiel einer erfindungsgemäßen Strahleranordnung, die besonders kompakte Geräteabmessungen ermöglicht. Die in der Fig. 8 wiedergegebene Bestrahlungsvorrichtung 31 besteht aus der Reihenanordnung von fünf Strahlern. Es sind drei UV-Strahler 32, 33 (UV/IR-Mischstrahler) und zwei IR-Strahler 34 (Wärmestrahler) vorgesehen, die jeweils in Fassungen 35 eingeschraubt sind. Durch die Verwendung von UV/IR-Mischstrahlern 32, 33, diese sind mit einer Vorschaltglühwendel ausgestattet, kann auf ein zusätzliches Vorschaltgerät verzichtet werden. Die Strahler 32, 33, 34 weisen jeweils an der Kolbeninnenfläche eine nur die Kolbenkuppe 36 freilassende Reflektorbeschichtung 37 auf. Über die Fassungen 35 sind die Strahler 32, 33, 34 an einem langgestreckten und ebenen Trägerteil 38 angebracht. Der in der Mitte der Reihenanordnung befindliche UV-Strahler 32 — durch seine Strahlungsrichtung ist die Hauptstrahlungsrichtung vorgegeben — ist mit seiner Reflektorachse 39 zum Trägerteil 38 senkrecht ausgerichtet, während die beiden äußeren UV-Strahler 33 und die IR-Strahler 34 mit ihren Reflektorachsen 40 bzw. 41 jeweils gegenüber der Senkrechten 42 zum Trägerteil 38 (bzw. gegenüber der Hauptstrahlungsrichtung) geneigt angeordnet sind. Die Strahlergruppen 33, 34 zu beiden Seiten des mittleren Strahlers 32 sind mit ihren Reflektorachsen 40, 41 in jeweils entgegengesetzte Richtungen — nach außen — geneigt. Alle Reflektorachsen 39, 40, 41 liegen in einer Ebene; diese verläuft zum Trägerteil 38 senkrecht und durch dessen Längsachse. Die Bestrahlungsvorrichtung 31 ist für den waagerechten Betrieb vorgesehen. Die Bestrahlungsfläche (nicht gezeigt) befindet sich unterhalb des Gerätes — sie verläuft senkrecht zur Hauptstrahlungsrichtung. Das ebene Trägerteil 38 ist parallel zur Bestrahlungsfläche ausgerichtet. Dadurch ergibt sich zwischen den Strahlern 33, 34 und der Bestrahlungsfläche derselbe Neigungswinkel wie zwischen den Strahlern 33, 34 und dem Trägerteil 38. Der Neigungswinkel $\alpha$ ist bei allen Strahlern 33, 34 gleich, er beträgt 15°. Dabei liegt ein über die Reihenanordnung äquidistanter Strahlerabstand a (gemessen zwischen den Mitten der Kuppen 36 benachbarter Strahler) von etwa 25 cm vor; aufeinanderfolgende UV-Strahler 32, 33 haben somit einen Abstand von etwa 50 cm voneinander. Die Schrägstellung der Strahler 33, 34 wird durch entsprechend (keilförmig) ausgebildete Fassungsblöcke 35 erreicht. Das Trägerteil 38 ist Bestandteil eines die Strahler 32, 33, 34 aufnehmenden kastenförmigen Gehäuses 43. Das Trägerteil 38 bildet die Oberseite des Gehäuses 43, das noch aus den Längsseiten 44 und den Schmalseiten 45 sowie einem mit Aussparungen 46 versehenen Unterteil 47 besteht. Die Strahler 32, 33, 34 sind durch die Aussparungen 46 des Unterteils 47 durchgesteckt und in den Fassungen 35 eingesetzt. Die Strahlerkuppen 36, die die Strahlungsaustrittsflächen bilden, liegen frei.

In Fig. 9 ist die Bestrahlungsvorrichtung 31 von unten gezeigt. Die Strahler 32, 33, 34 ragen mit ihren Kuppen 36 über das das Geräteinnere abdeckende Unterteil 47 heraus; dabei sind die Aussparungen 46 weitestgehend verdeckt. Im Unterteil 47 sind noch Lüftungsöffnungen 48 vorgesehen. Die Gehäuselängsseiten 44 sind unmittelbar, seitlich von den Strahlerkuppen 36 ausgehend, senkrecht zum Trägerteil 38 hochgezogen. An der Rückseite der Bestrahlungsvorrichtung 31 ist ein Schwenkarm 49 zur Befestigung der Vorrichtung an der Wand (nicht gezeigt) vorgesehen. Der Haltearm 49 ist mittels des Gelenkes 50 an der hinteren Längsseite 44 des Gerätes befestigt. Am anderen Ende des Schwenkarmes 49 befindet sich das Gelenk 51. Dieses ist an der Halteplatte 52 angebracht, die an der Wand befestigt wird.

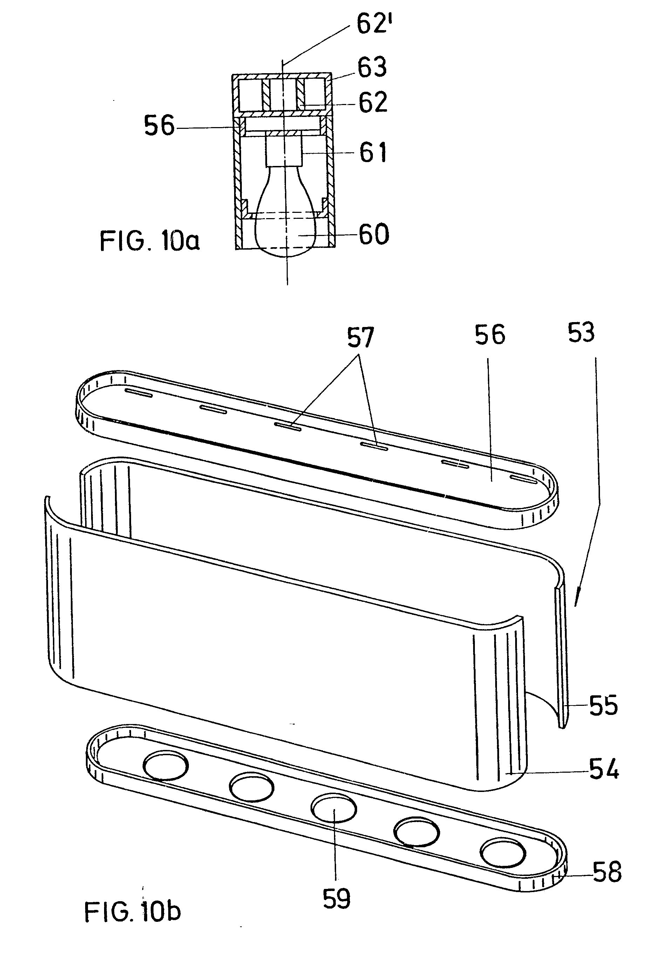

Die Fig. 10a bis 13a und 10b bis 13b zeigen verschiedene Gehäusekonstruktionen, die kostengünstig herstellbar sind. Dabei wurde der Übersichtlichkeit halber bei den perspektivischen Darstellungen auf der Wiedergabe der Strahler und ihrer Fassungen verzichtet.

Die Fig. 10a und 10b zeigen den Aufbau eines Gehäuses 53, wie es etwa den Fig. 1 bis 9 entspricht. Es besteht aus zwei formgleichen Schalen 54 und 55 für die Seitenwände, aus einem einsetzbaren Deckel 56 mit Lüftungsschlitzen 57 und einem einsetzbaren Boden 58 mit Öffnungen 59 für die Strahler 60. Die Strahlerfassungen 61 sind am Deckel 56 befestigt. Dieser Aufbau kann aus Blech hergestellt und verschweißt, vernietet oder verschraubt sein. Das Gelenk 62 (mit Achse 62') für den Haltearm 63 kann gemäß Fig. 10a oben, aber auch ohne Schwierigkeiten seitlich befestigt werden, wobei das Blech genügend stabil sein muß. Eine Herstellung dieser Gehäuseform aus Kunststoff ist ebenfalls möglich.

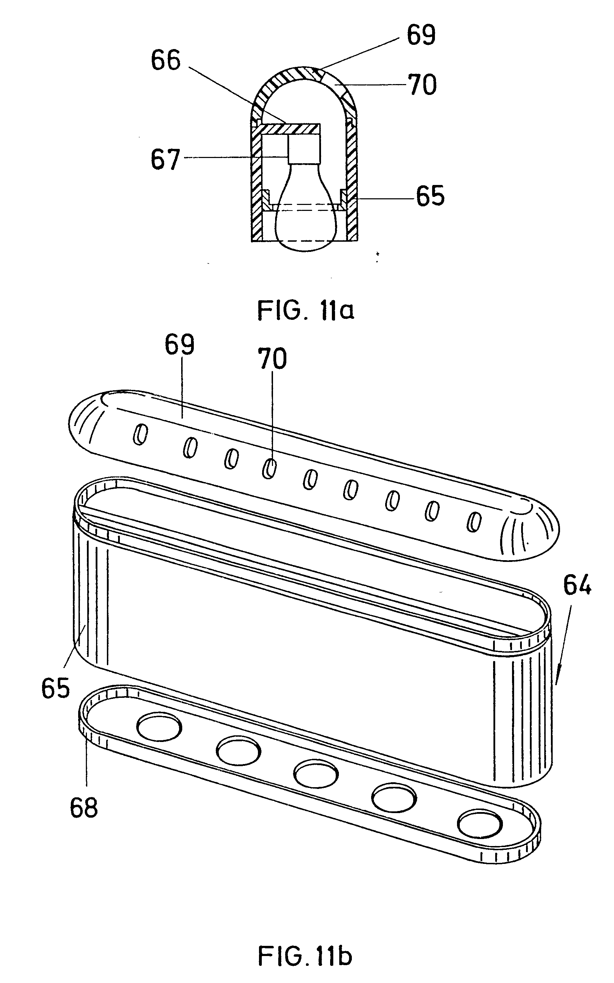

Die Fig. 11a und 11b zeigen ein aus Kunststoff gespritztes Gehäuse 64 mit einem einstückigen Mantel 65, an den innenseitig eine Halteleiste 66 für die Strahlerfassungen 67 angespritzt ist. Sie kann aber auch ebenso wie der Boden 68 eingeklebt werden. Auf den Mantel 65 kann ein flacher Deckel wie nach Fig. 10 auf- bzw. in ihn eingelegt werden. Sofern das Gelenk an der Seitenwand angeordnet wird, kann auch eine abgerundete einstückige Haube 69 mit Luftschlitzen 70 aufgesetzt werden, wie sie in den Fig. 11a und 11b dargestellt ist.

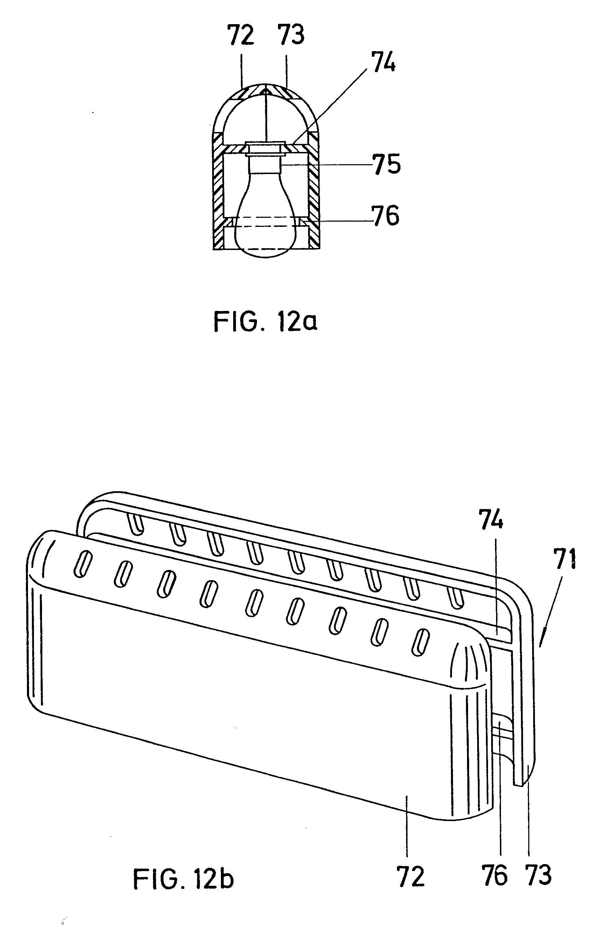

Bei der Ausführungsform gemäß Fig. 12a und 12b ist das ganze Gehäuse 71 aus zwei Schalenhälften 72 und 73 aufgebaut, an die der Träger 74 für die Strahlerfassungen 75 ebenso angeformt ist wie der Boden 76. Da beide Schalenhälften gleich sind, ist zur Herstellung nur ein Werkzeug erforderlich. Auch bei dieser Ausführungsform kann statt des gewölbten oberen Abschlusses ein flacher Abschluß vorgesehen werden, so daß der Schwenkarm auch oben befestigt werden kann.

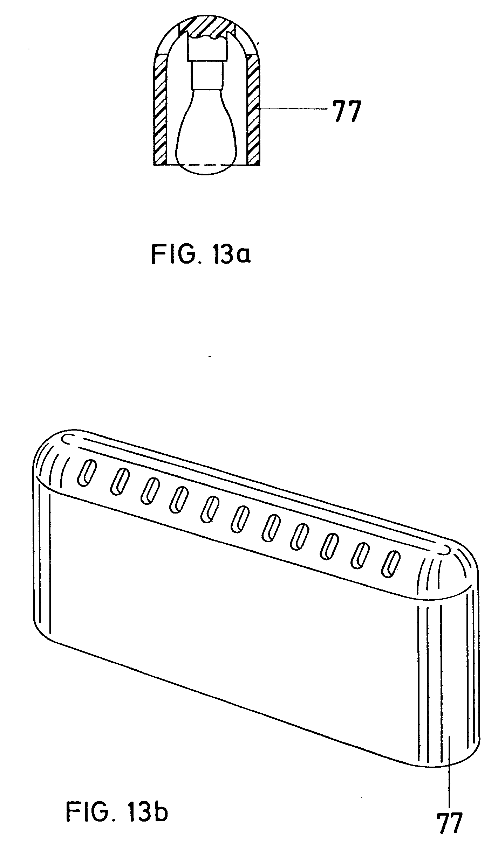

Das gleiche gilt für das Ausführungsbeispiel nach Fig. 13a und 13b. Das Gehäuse 77 ist einstückig aus Kunststoff gespritzt. Dabei ist das Anspritzen des Trägers für die Strahlerfassungen nur an der Gehäusewand direkt möglich, sofern keine komplizierten Werkzeuge verwendet werden sollen. Sofern ein Boden gewünscht wird, muß er von unten eingesetzt werden.

Allen beschriebenen Ausführungsformen ist gemeinsam, daß sie einfach und kostengünstig in Massenfertigung hergestellt werden können. Da die Gehäuse — insbesondere die aus Kunststoff — verhältnismäßig leicht sind, ist eine entsprechend leichte Ausführung des Haltearmes möglich. Durch die Kaminwirkung wird bei den beschriebenen Gehäusen eine hinreichende Kühlung der Strahler erzielt.

Die Befestigung des Schwenkarmhalters an der Wand hat selbstverständlich den an die Stabilität der Anordnung zu stellenden Anforderungen zu genügen. Dazu kann es erforderlich sein, die Basis der Halterung für Wände geringer Festigkeit zu vergrößern. Das ist durch Hinterlegen einer Metallplatte und Verankern derselben in der Wand möglich.

Ferner kann selbstverständlich der elektrische Anschluß auch über ein Kabel an einer Steckdose erfolgen, sofern eine feste Installation nicht erwünscht bzw. möglich ist.

**Patentansprüche**

1. Bestrahlungsvorrichtung, insbesondere Heimsolarium, mit einem Gehäuse, das mehrere — mindestens drei — jeweils mit einem Reflektor versehene UV- und/oder IR-Strahler enthält, die an einem Trägerteil des Gehäuses angebracht sind, wobei durch die Strahlungsrichtung eines oder mehrerer Strahler eine vertikale Hauptstrahlungsrichtung vorgegeben ist, und mit einer Haltevorrichtung, bestehend aus mindestens einem langgestreckten Haltearm, der an seinen Enden jeweils mit einem Gelenk versehen ist, wobei der Haltearm mittels des ersten Gelenkes am Gehäuse befestigt ist, dadurch gekennzeichnet, daß die Achsen der beiden Gelenke des Haltearmes im wesentlichen parallel zur Hauptstrahlungsrichtung verlaufen, wobei das zweite Gelenk an einer Halteplatte zum Befestigen der Bestrahlungsvorrichtung gelagert ist, und daß die Strahler innerhalb des Gehäuses in einer einzigen Reihe angeordnet sind.

2. Bestrahlungsvorrichtung nach Anspruch 1, dadurch gekennzeichnet, daß die vertikale Hauptstrahlungsrichtung durch die Strahlungsrichtung eines mittleren Strahlers oder einer mittleren Strahlergruppe vorgegeben ist und die außermittigen Strahler mit ihren Reflektorachsen gegenüber der Hauptstrahlungsrichtung geneigt sind.

3. Bestrahlungsvorrichtung nach Anspruch 2, dadurch gekennzeichnet, daß die Reflektorachsen aller Strahler im wesentlichen in einer Ebene liegen und die Neigung der Reflektorachsen — bei den außermittigen Strahlern — mit der Richtung übereinstimmt, in der der betreffende Strahler oder die Strahlergruppe gegenüber dem mittleren Strahler oder der mittleren Strahlergruppe versetzt ist.

4. Bestrahlungsvorrichtung nach Anspruch 1 und 2, dadurch gekennzeichnet, daß das Trägerteil für die Strahler langgestreckt und im wesentlichen eben ist und die Hauptstrahlungsrichtung senkrecht zum Trägerteil verläuft.

5. Bestrahlungsvorrichtung insbesondere nach Anspruch 1, dadurch gekennzeichnet, daß das erste Gelenk — zwischen Haltearm und Strahlergehäuse — an einem Ende des Gehäuses an dessen Ober- oder Rückseite angeordnet ist.

6. Bestrahlungsvorrichtung insbesondere nach Anspruch 1, dadurch gekennzeichnet, daß zwei Haltearme vorgesehen sind, die jeweils an den Enden des Strahlergehäuses an dessen Ober- oder Rückseite befestigt sind, und daß die einzelnen Haltearme in ihrer Länge unterteilt und die zusammengehörigen Teilarme durch ein (mittleres) Gelenk miteinander verbunden sind, dessen Achse zu den übrigen Gelenkachsen parallel liegt.

7. Bestrahlungsvorrichtung insbesondere nach Anspruch 2 und 3, dadurch gekennzeichnet, daß der Neigungswinkel der mit ihren Reflektorachsen geneigten Strahler oder Strahlergruppen jeweils 10° bis 30° beträgt.

8. Bestrahlungsvorrichtung insbesondere nach Anspruch 2, 3 und 7, dadurch gekennzeichnet, daß die Reflektorachsen aller außermittigen Strahler bzw. Strahlergruppen denselben Neigungswinkel aufweisen.

9. Bestrahlungsvorrichtung insbesondere nach Anspruch 2, 3 und 7, dadurch gekennzeichnet, daß die Neigungswinkel der außermittigen Strahler bzw. Strahlergruppen mit zunehmendem Abstand von der Mitte größer werden.

10. Bestrahlungsvorrichtung insbesondere nach Anspruch 2 und 3, dadurch gekennzeichnet, daß bei der Reihenanordnung UV-Strahler (bzw. UV/IR-Mischstrahler) und IR-Strahler abwechselnd angeordnet sind.

11. Bestrahlungsvorrichtung insbesondere nach Anspruch 2 und 3, dadurch gekennzeichnet, daß die einzelnen Strahler bzw. Strahlergruppen in etwa gleichen Abständen zueinander angeordnet sind.

12. Bestrahlungsvorrichtung insbesondere nach Anspruch 2 und 3, dadurch gekennzeichnet, daß der Neigungswinkel der Reflektorachsen der außermittigen Strahler mittels einer Rastvorrichtung veränderlich einstellbar ist.

13. Bestrahlungsvorrichtung nach Anspruch 1 bis 12, dadurch gekennzeichnet, daß die Strahler im wesentlichen bis auf ihre Strahlungsaustrittsflächen vom Gehäuse umgeben sind.

## Claims

1. An irradiation device, especially a solarium for the home, comprising a housing with several — at least three — UV and/or IR emitting radiation sources, each of them provided with a reflector, which are mounted to a carrier member of the housing, with a main direction of radiation emission being defined by the direction of radiation emission of one or several of the radiation sources, and a support device, wherein at least one support arm is pivotally affixed to the housing by a (first) joint whose axis is essentially parallel to the main direction of radiation emission and the other end of the support arm comprises a further (second) joint whose axis is parallel to that of the first joint and which is supported on a carrier plate for affixing the irradiation device.

2. An irradiation device as claimed in claim 1, wherein the main direction of radiation emission is defined by the direction of radiation emission of a central radiation source or of a central group of radiation sources, and wherein further radiation sources are arranged in a row with the central radiation source or the central group of radiation sources, with the eccentrical radiation sources having the reflector axes inclined relative to the main direction of radiation emission.

3. An irradiation device as claimed in claim 2, wherein the reflector axes of all the radiation sources lie substantially in one plane and the inclination of the reflector axes — in the case of the eccentrical radiation sources — coincides with the direction in which the respective radiation source or the respective group of radiation sources is displaced relative to the central radiation source or to the central group of radiation sources.

4. An irradiation device as claimed in claims 1 and 2, wherein the carrier member for the radiation sources is elongate and substantially plane and the main direction of radiation emission is perpendicular to the carrier member.

5. An irradiation device, especially as claimed in claim 1, wherein the first joint — between the support arm and the housing of the radiation sources — is arranged at one end of the housing at the upper side or at the back side thereof.

6. An irradiation device, especially as claimed in claim 1, wherein two support arms are provided which in each case are attached to the ends of the housing of the radiation sources at the upper side or at the back side thereof and wherein the individual support arms are divided over their length and the parts of the arms which belong together are connected with one another by a (middle) joint whose axis is parallel to the other joint axes.

7. An irradiation device, especially as claimed in claims 2 and 3, wherein the angle of inclination of the radiation sources or groups of radiation sources having inclined reflector axes is in each case from 10° to 30°.

8. An irradiation device, especially as claimed in claims 2, 3 and 7, wherein the reflector axes of all the eccentrical radiation sources or groups of radiation sources have the same angle of inclination.

9. An irradiation device, especially as claimed in claims 2, 3 and 7, wherein the angles of inclination of the eccentrical radiation sources or groups of radiation sources become larger as the distance from the center increases.

10. An irradiation device, especially as claimed in claims 2 and 3, wherein in the row arrangement UV sources (or blended UV/IR sources) and IR sources alternate.

11. An irradiation device, especially as claimed in claims 2 and 3, wherein the individual radiation sources or groups of radiation sources are about equidistant from one another.

12. An irradiation device, especially as claimed in claims 2 and 3, wherein the angle of inclination of the reflector axes of the eccentrical radiation sources may be adjusted by means of a click-stop device.

13. An irradiation device as claimed in claims 1 to 12, wherein the radiation sources with the exception of the radiation emission areas are substantially encased by the housing.

**Revendications**

1. Installation d'irradiation, notamment solarium à domicile, comportant un boîtier (1) contenant plusieurs — au moins trois — émetteurs (4, 5; 32, 33, 34) de rayonnement ultraviolet et/ou de rayonnement infrarouge comportant chacun un réflecteur et qui sont montés sur un élément de support (38) du boîtier, et dans lequel une direction verticale de rayonnement principal est prédéterminée par la direction de rayonnement d'un ou de plusieurs émetteurs de rayonnement (4, 5; 32, 33, 34) et comportant un dispositif de support (2, 3; 12, 16) constitué par au moins un bras de support (2; 12) dont les extrémités sont munies d'articulations respectives (6, 8, 14, 15) et qui est fixé au moyen de la première articulation (8, 16) sur le boîtier, caractérisée par le fait que les axes (6', 8') des deux articulations (6, 8, 14, 15) du bras de support (2, 12), sont essentiellement parallèles à la direction de rayonnement principal (7, 39), la seconde articulation (6, 14) est montée sur une plaque de support permettant la fixation de l'installation d'irradiation et les émetteurs de rayonnement (4, 5, 32, 33, 34) sont disposés à l'intérieur du boîtier (1) suivant une rangée unique.

2. Installation d'irradiation selon la revendication 1, caractérisée en ce que la direction verticale de rayonnement principal (7, 39) est prédéterminée par la direction de rayonnement d'un émetteur de rayonnement central (4, 32) ou d'un groupe central d'émetteurs de rayonnement et les axes (40, 41) des réflecteurs des émetteurs de rayonnement non situés au centre sont inclinés par rapport à la direction de rayonnement principal (7, 39).

3. Installation d'irradiation selon la revendication 2, caractérisée en ce que les axes (39, 40, 41) des réflecteurs de tous les émetteurs de rayonnement sont situés essentiellement dans un plan et l'inclinaison des axes des réflecteurs (40, 41) dans le cas des émetteurs de rayonnement non situés au centre-coïncide avec la direction suivant laquelle l'émetteur considéré ou le groupe d'émetteurs est décalé par rapport à l'émetteur central (32) ou par rapport au groupe central d'émetteurs.

4. Installation d'irradiation selon les revendications 1 et 2, caractérisée par le fait que l'élément de support (38) pour les émetteurs de rayonnement (32, 33, 34) est allongé et essentiellement plan et la direction de rayonnement principal (39) est perpendiculaire à l'élément de support (38).

5. Installation d'irradiation selon la revendication 1, caractérisée en ce que la première articulation — située entre le bras de support (2, 12) et le boîtier (1) contenant les émetteurs — est disposée à une extrémité du boîtier (1) sur la face supérieure ou la face arrière de ce dernier.

6. Installation d'irradiation selon la revendication 1, caractérisée en ce qu'il est prévu deux bras de support (18, 26) qui sont fixés respectivement aux extrémités du boîtier (19, 29) contenant les émetteurs, au niveau de la face supérieure ou de la face arrière de ce dernier, et que les différents bras de support (18, 26) sont subdivisés suivant leur longueur et les bras partiels (23, 24, 27, 28) réunis ensemble sont reliés entre eux par l'intermédiaire d'une articulation (médiane) (25) dont l'axe est parallèle aux autres axes (20, 21) des articulations.

7. Installation d'irradiation selon les revendications 2 et 3, caractérisée en ce que l'angle ($\alpha$) d'inclinaison des émetteurs de rayonnement ou des groupes d'émetteurs de rayonnement (33, 34), dont les axes des réflecteurs sont inclinés, est égal à une valeur comprise entre 10 et 30°.

8. Installation d'irradiation selon les revendications 2, 3 et 7, caractérisée en ce que les axes des réflecteurs de tous les émetteurs de rayonnement ou de tous les groupes d'émetteurs de rayonnement décalés par rapport au centre (33, 34) possèdent le même angle d'inclinaison ($\alpha$).

9. Installation d'irradiation selon les revendications 2, 3 et 7, caractérisée en ce que les angles d'inclinaison des émetteurs de rayonnement ou des groupes d'émetteurs de rayonnement décalés par rapport au centre (33, 34) augmentent au fur et à mesure qu'augmente la distance par rapport au centre.

10. Installation d'irradiation selon les revendications 2 et 3, caractérisée en ce que dans le cas d'une disposition des émetteurs de rayonnement (4, 5) selon une rangée, des émetteurs de rayonnement ultraviolet (ou des émetteurs mixtes de rayonnement ultraviolet/infrarouge (4) et des émetteurs de rayonnement infrarouge (5) sont disposés de façon alternée.

11. Installation d'irradiation selon les revendications 2 et 3, caractérisée en ce que les différents émetteurs de rayonnement ou les différents groupes d'émetteurs de rayonnement (4, 5; 32, 33, 34) sont disposés à des distances réciproques approximativement identiques.

12. Installation d'irradiation selon les revendications 2 et 3, caractérisée en ce que l'angle ($\alpha$) d'inclinaison des axes des réflecteurs des émetteurs de rayonnement (33, 34) décalés par rapport au centre peut être réglé de façon variable au moyen d'un dispositif d'encliquetage.

13. Installation d'irradiation selon les revendications 1 à 12, caractérisée en ce que les émetteurs de rayonnement sont entourés par le boîtier essentiellement jusqu'au niveau de leurs surfaces de sortie du rayonnement.

FIG.1

FIG. 2

FIG. 3

FIG. 4

FIG. 5

FIG. 6

FIG. 7

FIG.8

FIG. 9

FIG. 10a

FIG. 10b

FIG. 11a

FIG. 11b

72  73

74

75

76

FIG. 12a

74

71

FIG. 12b       72       76  73

FIG. 13a

FIG. 13b